(19) Europäisches Patentamt
European Patent Office
Office européen des brevets

(11) Publication number: 0 149 924 B1

# (12) EUROPEAN PATENT SPECIFICATION

(45) Date of publication of patent specification: 14.03.90

(51) Int. Cl.$^5$: A 61 K 6/08, C 08 F 259/08 // (C08F259/08, 220:22)

(21) Application number: 84309162.0

(22) Date of filing: 31.12.84

## (54) Composition for lining a denture base.

(30) Priority: 05.01.84 JP 394/84

(43) Date of publication of application:
31.07.85 Bulletin 85/31

(45) Publication of the grant of the patent:
14.03.90 Bulletin 90/11

(84) Designated Contracting States:
DE FR GB

(56) References cited:
GB-A- 982 814
GB-A-2 027 043
GB-A-2 132 209

CHEMICAL ABSTRACTS, vol. 102, no. 2, January 1985, page 367, abstract no. 12439n, Columbus, Ohio, US; & JP-A-59 144 708 (KUREHA CHEMICAL INDUSTRY CO., LTD) 18-08-1984

(73) Proprietor: KUREHA KAGAKU KOGYO KABUSHIKI KAISHA
9-11 Horidome-cho 1-chome Nihonbashi Chuo-ku
Tokyo 103 (JP)

(73) Proprietor: Masuhara, Eiichi
2-5-10 Hon-Komagome
Bunkyo-ku Tokyo (JP)

(73) Proprietor: Hayakawa, Iwao
2-17-10 Sannoh
Ohta-ku Tokyo (JP)

(72) Inventor: Masuhara, Eiichi
2-5-10 Hon-Komagome
Bunkyo-ku Tokyo (JP)
Inventor: Hayakawa, Iwao
2-17-15-Sannoh
Ohta-ku Tokyo (JP)
Inventor: Bannai, Nobuo
33 Shakadoh, Nishiki-machi
Iwaki-shi Fukushima-ken (JP)
Inventor: Yasumi, Hideyuki
78-96 Hananoi Nishiki-machi
Iwaki-shi Fukushima-ken (JP)

(74) Representative: Woods, Geoffrey Corlett et al
J.A. KEMP & CO. 14 South Square Gray's Inn
London WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 149 924 B1

**Description**

The present invention relates to a composition for lining a denture base.

Various materials have been studied for lining the inner surface of a denture base in order to moderate the occlusal pressure acting on the denture base and to transfer the moderated pressure to the mucous membrane surface in the oral cavity. However, no adequate material for that purpose has been found.

In order to solve the problem, the present inventors proposed a denture base lined with a fluoropolymer (JP—A—80021919). The fluoropolymer scarcely absorbs water, is excellent in adhesiveness to the base material of the denture and in use does not deteriorate for a long time (longer than 2 years). However, the fluoropolymer is more complicated to apply to a denture base than commercialized materials and some problems still remain as regards its softness.

In order to solve these problems, the present invention have further proposed lining a denture base with a soft material prepared by mixing the above-mentioned fluoropolymer with a fluorine-containing monomer obtained by bringing a fluorine-containing telomer having a molecular weight of not less than 1500 into esterification with acryloyl chloride or methacryloyl chloride and kneading the thus formed mixture (JP—A—84144708). Although the soft material is an excellent material for moderating and transferring the occlusal pressure on the mucous membrane surface in the oral cavity or for stabilizing the denture base during a relatively short period of utilization in the oral cavity, the following problem remains where use for longer than one year is desired.

Namely, since the material has been softened by mixing the fluoropolymer with the fluorine-containing monomer, although the processability of the lining material is remarkably improved, the softness of the material does not change after being lined onto the inner surface of the denture base and during the use of the thus lined denture base.

Accordingly, flow of the thus lined material is induced by occlusal pressure resulting in the gradual reduction of the thickness of the layer of the lined material and then finally in the disappearance of the layer.

The present inventors have further studied in order to solve this problem and provide a soft material having a fluidity in the processing stage for lining and having an ideal hardness and elasticity after being applied as the lining.

The present invention provides a composition suitable for use in lining a denture base, which composition comprises from 3 to 30 parts by weight of a fluorine-containing copolymer, from 3 to 30 parts by weight of a ($C_1$ to $C_{12}$-alkyl)acrylate or methacrylate and from 0.1 to 3.0 parts by weight of a polymerization-initiator per 100 parts by weight of a monomer represented by the formula (I):

$$CH_2{=}\underset{\displaystyle R^1}{\underset{|}{C}}{-}COOR^2 \qquad (I)$$

wherein $R^1$ represents a hydrogen atom or a methyl group and $R^2$ represents the group formed by removing a hydroxyl group from a hydroxyl group-containing and fluorine-containing telomer having a molecular weight of from 200 to 1500.

The composition of the present invention (hereinafter referred to as the present material) is soft and putty-like and is suitable for use in lining the inner surface of a denture base according to a conventional method.

The present material may, for example, be applied to a doughy, rubbery methacrylic resin shaped in a gypsum mold and, while applying pressure thereto by another gypsum mold, the thus pressed material is heated and thereby polymerized to obtain a denture base which is made of the methacrylic resin and has the inner surface thereof lined with a soft elastic copolymer.

The present material has excellent processability, and does not require a particular method to prepare a denture base because it is very soft.

The term "putty-like" in the present invention includes materials ranging from those which are very viscous and sticky to those which are putty-like, and which have a penetration resistance of from 5 to 50 $g/mm^2$. In addition, after polymerization the shore hardness of the surface of the thus lined material on the under surface of the denture base is preferably from 10 to 30 (Shore D type, at 25°C) and the lined material after polymerization has a modulus of rigidity of 5 to 50 $kg/cm^2$, an adhesion strength to the surface of the denture base of from 40 to 100 $kg/cm^2$ (fracture of specimen occurs) and a water absorption of less than 0.2%.

The denture base lined with the present material moderates the occlusal pressure and transfers the pressure to the mucous membrane surface in the oral cavity, and has good conformity between the denture base and the gingiva. Furthermore, a denture base lined with the present material has remarkable durability, showing no deterioration after use for more than two years in an oral cavity.

The monomer represented by the formula (I) can be produced according to the method disclosed in JP—A—84117503. A telomer is produced by subjecting a mixture of a fluorine-containing monomer as a taxogen and a primary alcohol for example methanol as a telogen to telomerization in an autoclave in the presence of an organic peroxide such as diacyl peroxide or dialkyl peroxydicarbonate at a temperature of

from 10 to 150°C, preferably from 40 to 50°C, under an initial pressure of from 15 to 30 kg/cm$^2$, preferably from 20 to 28 kg/cm$^2$, for from 8 to 12 hours, preferably from 8 to 12 hours. The molar ratio of the telogen to the taxogen has a large influence on the molecular weight of the telomer obtained, and in order to have a telomer having a molecular weight of from 200 to 1500, preferably from 800 to 1200, the molar ratio is preferably from 3:1 to 7:1. If the ratio is below 3, the molecular weight of the telomer becomes so large that a solid telomer is formed rather than the preferred oily telomer. If the ratio is over 7, the yield per telomerization batch is lowered which is disadvantageous.

As the taxogen, a mixture of at least two monomers selected from vinylidene fluoride, vinyl fluoride, trifluoroethylene, chlorotrifluoroethylene, tetrafluoroethylene and hexafluoropropylene is preferred, particularly a mixture comprising not less than 50% by weight of vinylidene fluoride. For a telomer comprising vinylidene fluoride as the main component, the lining material has a more suitable pliability, and an excellent durability is more easily obtained than in other cases.

In consideration of the telomerization pressure, the reaction is preferably carried out at a temperature of from 40 to 50°C in the presence of dialkyl peroxydicarbonate initiator for from 8 to 10 hours.

After the telomerization is over, the reaction mixture is collected and the telogen distilled out to obtain a colourless, transparent and viscous liquid telomer.

The thus obtained telomer is esterified to obtain the monomer represented by the formula (I) as follows.

After dissolving the telomer in a solvent such as carbon tetrachloride or chloroform, acrylic chloride or methacrylic chloride is added to the solution dropwise at a temperature of from 0 to 100°C in from 30 to 60 min. The esterification time is from about 30 to 180 min. It is preferred to add a tertiary amine such as triethylamine to fix the by-product hydrogen chloride in the reaction system. The molar amount of the tertiary amine is preferably about 110% of that of the acid chloride.

Since the ester moiety of the monomer represented by the formula (I) consists of the above-mentioned telomer, the present material exhibits, after it has been polymerized and solidified, an appropriate softness and an excellent durability.

The fluorine-containing copolymer is preferably derived from two or more fluoro-olefinic monomers having 2 to 3 carbon atoms, preferably selected from vinylidene fluoride, vinyl fluoride, trifluoroethylene, chlorotrifluoroethylene, tetrafluoroethylene and hexafluoropropylene. The rigidity of the fluorine-containing copolymer is preferably from 10 to 150 kg/cm$^2$. The preferred fluorine-containing copolymer is a terpolymer of from 40 to 60% by weight of vinylidene fluoride and from 20 to 30% by weight of each of two of tetrafluoroethylene, chlorotrifluoroethylene and hexafluoropropylene.

Of the ($C_1$ to $C_{12}$-alkyl) acrylates or methacrylates, those having an alkyl group containing from 1 to 8 carbon atoms are preferred. Methyl acrylate, methyl methacrylate, ethyl acrylate, ethyl methacrylate, propyl acrylate, propyl methacrylate, butyl acrylate, butyl methacrylate, octyl acrylate, octyl methacrylate, 2-ethylhexyl acrylate and 2-ethylhexyl methacrylate may be mentioned.

As the polymerization initiator organic peroxides having a long half life at room temperature such as benzoyl peroxide and di-t-butyl peroxide are preferred.

The amount of the fluorine-containing copolymer in the present material is from 3 to 30 parts by weight, preferably from 5 to 20 parts by weight, per 100 parts by weight of the monomer represented by the formula (I). The weight ratio is selected from the view points of (1) giving plasticity to the putty-like mixture for facilitating the processing operation and (2) giving strength to the layer of the solidified lining material after polymerization. When the amount of the flourine-containing copolymer is less than 3 parts by weight, the viscosity of the putty-like material is so low that processing thereof is a little difficult, and the strength of the solidified lining material after polymerization is also low.

On the other hand, when the amount of the fluorine-containing copolymer is greater than 30 parts by weight since the viscosity of the putty-like mixture is too high, the processing thereof is difficult; the strength of the solidified lining material after polymerization is high.

The amount of the ($C_1$ to $C_{12}$-alkyl) acrylate or methacrylate in the present material is from 3 to 30 parts by weight, preferably from 5 to 20 parts by weight per 100 parts by weight of the monomer represented by the formula (I). The purpose of the alkyl acrylate or methacrylate is to disperse the polymerization initiator uniformly in the putty-like mixture and to adjust the strength and the hardness of the solidified lining material after polymerization. When the amount of the alkyl acrylate or methacrylate is less than 3 parts by weight, the polymerization initiator is not uniformly dispersed. On the other hand, when the amount is greater than 30 parts by weight, although the strength of the solidified lining material after polymerization is extremely high, the hardness thereof is too large for it to be used as a soft lining material.

In addition, in order to adjust the hardness and the strength of the layer of the lining material after polymerization, a cross-linking agent such as triethyleneglycol dimethacrylate may be added before polymerization.

The present material is prepared as follows.

After adding the fluorine-containing copolymer to the monomer represented by the formula (I), the mixture is kneaded at ambient temperature or at an elevated temperature, preferably at from 120 to 150°C for carrying out the kneading effectively. When the mixture is heated for kneading, the kneaded mixture is cooled to room temperature. After adding the alkyl acrylate or methacrylate containing the polymerization

initiator dissolved therein to the kneaded mixture, the thus obtained mixture is well kneaded at room temperature to obtain the present material.

The present invention also provides, a method of lining a denture base, which method comprises lining the denture base with the present material and polymerizing the material by heating.

In preparing a denture base the present material may be stretched and pressure-welded, using a doctor blade or fingers, onto the surface of a doughy, rubbery acrylic resin shaped by a gypsum mold, as in a conventional method, and the surface facing the mucous membrane surface, the present material and the acrylic resin are pressed together as in a conventional method, and polymerized by heating. The monomer represented by the formula (I) polymerizes to give a denture base having the inner surface lined with a soft elastic resinous material.

The present invention is explained in more detail in the following non-limitative Examples.

Example 1:

1) Preparation of a fluorine-containing telomer:

After introducing 12 g of di-n-propyl peroxydicarbonate and 2,370 g (74.06 mol) of methanol into a 6-litre stainless steel autoclave and replacing the air in the autoclave by gaseous nitrogen, the autoclave was cooled to about −30°C in a methanol-dry ice bath, and 597 g (9.33 mol) of vinylidene fluoride, 358 g (3.07 mol) of chlorotrifluoroethylene and 239 g (2.39 mol) of tetrafluoroethylene were introduced thereinto (the weight ratio of the liquefied monomers being 50:30:20 respectively). After closing the autoclave, the resultant mixture was heated to 40°C to initiate polymerization. The reaction was carried out for 8 hours at the same temperature under stirring. With the progress of polymerization, the inner pressure of the autoclave reduced from 24 kg/cm$^2$ to 4 kg/cm$^2$.

Then, the reaction was discontinued and the residual gases in the autoclave were released. The thus formed reaction mixture was distilled, thereby recovering methanol and obtaining a transparent oily substance.

The thus obtained oily substance was washed with water at 90 to 100°C to decompose and remove the residual organic peroxide, and after separation of the oily substance, it was distilled under a reduced pressure of 20 mmHg (2.7 kPa), thereby obtaining 1,027 g of a colourless and transparent fluorine-containing telomer having a molecular weight of 1,050 and a viscosity of 7,500 cps (7.5 Pas) at 25°C in a yield of 86%.

2) Preparation of a monomer represented by the formula (I):

Into a round-bottomed flask provided with a stirrer, a reflux condenser and a dropping funnel, 12.6 g of methacryloyl chloride, 50 g of carbon tetrachloride, 100 g of the fluorine-containing telomer prepared in 1) and 15 g of triethylamine were introduced.

While keeping a water bath in which the flask was placed at a temperature of 80°C, the solution of methacryloyl chloride in carbon tetrachloride in the dropping funnel was added to the solution of the telomer under stirring in the flask drop by drop in 40 min. After stirring the mixture in the flask further for 30 min, 100 ml of water were added to the flask to dissolve, the thus precipitated triethylamine hydrochloride, and stirring was continued for 10 min. After cooling the reaction mixture and collecting the thus separated oily layer, carbon tetrachloride was completely distilled off from the oily layer by steam distillation followed by distillation at 20 mmHg (2.7 kPa) to obtain 102 g of the monomer represented by the formula (I) wherein $R^1$ is a methyl group and $R^2$ is a residual group formed by removing a hydroxyl group from the hydroxyl group-containing and fluorine-containing telomer comprising vinylidene fluoride, chlorotrifluoroethylene and tetrafluoroethylene in a weight ratio of 50:30:20 respectively having a molecular weight of 1050.

3) Preparation of a soft, putty-like material for lining a denture base

In a mortar, 100 g of the monomer obtained in 2) and 15 g of a soft copolymer of vinylidene fluoride, chlorotrifluoroethylene and tetrafluoroethylene in a weight ratio of 50:30:20 respectively, which has a rigidity of 67 kg/cm$^2$, were mixed and the resultant mixture was heated in a thermostat at 160°C. At the time when the temperature of the mixture became 150°C, it was taken out of the thermostat and well kneaded for about 10 min, followed by cooling to room temperature.

Thereafter, a solution of 1 g of benzoyl peroxide in 15 g of methyl methacrylate was added to the thus cooled mixture, and the thus prepared mixture was kneaded at 25°C (room temperature at the time). The kneading was carried out from the first state of a mixture of a liquid and a semi-solid to the final, uniform putty state as a mutual fusion of the two components, thereby obtaining a soft, putty-like material for lining a denture base. The interstitial resistance of the thus obtained present material (the hardness) was 7.5 g/mm$^2$.

4) Preparation of a denture base lined with the soft, putty-liky material according to the present invention:

The thus obtained soft, putty-like material was applied in a conventional process for preparing a denture base made of acrylic resin and lined with the present material. Namely, the soft, putty-like material shaped in a form of a ribbon about 10 cm in length, about 1.5 cm in width and 0.4 cm in thickness was lined on the inner surface of a denture base made of an acrylic resin by pressure while stretching the material by a doctor blade or fingers, and then the thus lined material was heated together with the denture base to

100°C, thereby causing polymerization of the monomers in the soft, putty-like material to obtain a denture base lined with the elastic and soft resin.

5) Determination of the elastic and soft resin lining

After shaping the above-mentioned mixture of the monomer, a soft copolymer of vinylidene fluoride, chlorotrifluoroethylene and tetrafluoroethylene, methyl methacrylate and the polymerization initiator and polymerizing the mixture between gypsum molds to form test pieces 36 mm in diameter and 7 mm in thickness, the Shore hardness (D) of the test piece was determined, the result being 23.

As a result of applying the denture base lined with the soft, putty-like material, which was prepared in 4), in an oral cavity of a 76 year old man no deterioration of the denture base was observed even after use for 2 years, and continued use of the denture base was possible.

The penetration resistance of the present material

After introducing 9 g of a test material in a compression cell of a tensilon tester and adjusting the temperature of the test material to 20°C, a stainless-steel 2.3 mm diameter rod was forced into the test material at a velocity of 100 mm/min, the level of interstitial resistance being recorded in g/mm$^2$ units.

Examples 2 to 9 and comparative examples 1 to 3

While using the same monomer represented by the formula (I) prepared in 2) of Example 1, soft, putty-like materials were prepared by changing the ratio of mixing thereof with the fluorine-containing copolymer and methyl methacrylate as shown in Table 1. The hardness of the thus obtained materials before and after polymerization is also shown in Table 1.

As is seen from Table 1, the soft, putty-like material of the present invention has an adequate softness and processability for lining, and after polymerization exhibits ideal hardness and elasticity for use as the lining material for a denture base.

TABLE 1

| Example or comparative example | Mixing ratio A:B:C*1) | Hardness of | | Suitability2) for | |
|---|---|---|---|---|---|
| | | mixture (g/mm$^2$) | after polymerization (Shore D) | processing | as lining material |
| Example 1 | 100:15:15 | 7.5 | 23 | A | A |
| 2 | 100:3:15 | 2.1 | 20 | B | A |
| 3 | 100:10:15 | 5.0 | 21 | A | A |
| 4 | 100:20:15 | 12.0 | 25 | A | A |
| 5 | 100:30:15 | 60.7 | 28 | B | B |
| 6 | 100:15:3 | 61.0 | 17 | B | A |
| 7 | 100:15:10 | 9.8 | 21 | A | A |
| 8 | 100:15:20 | 6.0 | 26 | A | A |
| 9 | 100:15:30 | 2.7 | 39 | B | B |
| Comparative example 1 | 100:1:15 | 0—1 | 19 | C | A |
| 2 | 100:40:15 | 240 | 32 | C | C |
| 3 | 100:15:40 | 1.0 | 45 | C | C |

Notes:

1) Mixing ratio A:B:C means weight ratio of the monomer represented by the formula (I): fluorine-containing copolymer: methyl methacrylate.

2) A: suitable, B: less suitable and C: bad.

Example 10

1) Preparation of a fluorine-containing telomer

In the same procedures as in 1) of Example 1 except for using hexafluoropropylene instead of tetrafluoroethylene, a telomer having a molecular weight of 980 comprising 50 parts by weight of vinylidene fluoride, 30 parts by weight of chlorotrifluoroethylene and 20 parts by weight of hexafluoropropylene was obtained.

2) Preparation of a monomer represented by the formula (I)

In the same procedures as in 2) of Example 1 except for using the fluorine-containing telomer prepared in 1) above, a monomer represented by the formula (I) wherein $R^1$ is a methyl group and $R^2$ is a residual group formed by removing a hydroxyl group from the hydroxyl group-containing and fluorine-containing telomer comprising 50 parts by weight of vinylidene fluoride, 30 parts by weight of chlorotrifluoroethylene and 20 parts by weight of hexafluoropropylene was obtained.

3) Preparation of a soft, putty-like material for lining a denture base

In the same procedure as in 3) of Example 1 except for using 100 g of the fluorine-containing monomer obtained in 2) above and 20 g of a soft copolymer comprising 50 parts by weight of vinylidene fluoride, 30 parts by weight of chlorotrifluoroethylene and 20 parts by weight of hexafluoropropylene having a rigidity of 62 kg/cm$^2$, and 10 g of methyl methacrylate, a soft, putty-like material according to the present invention was obtained, which had an interstitial resistance of 9.7 g/mm$^2$ before polymerization.

4) Preparation of a denture base lined with the soft, putty-like material according to the present invention

In the same manner as in 4) of Example 1 except for using the soft, putty-like material obtained in 3) above instead of the soft, putty-like material obtained in Example 1, the denture base lined with the elastic and soft resin was prepared.

5) Determination of the elastic and soft resin lining

The Shore hardness (D) of the test piece made of the lining material obtained by 4) above was determined as in 5) of Example 1 and the Shore D was 22.5.

As a result of applying the denture base lined with the soft, putty-like material obtained in 4) above in an oral cavity of a 74 year old man, no deterioration of the denture base was observed even after 2 years of use thereof, and continued use thereof was possible.

Example 11

1) Preparation of a fluorine-containing telomer

In the same procedures as in 1) of Example 1 except for using 180 g of tetrafluoroethylene and 418 g of hexafluoropropylene instead of 358 g of chlorotrifluoroethylene and 239 g of tetrafluoroethylene, a fluorine-containing telomer comprising 50 parts by weight of vinylidene fluoride, 15 parts by weight of tetrafluoroethylene and 35 parts by weight of hexafluoropropylene having a molecular weight of 940 was obtained.

2) Preparation of a monomer represented by the formula (I)

In the same procedures as in 2) of Example 1 except for using the fluorine-containing telomer prepared in 1) above, the monomer represented by the formula (I) wherein $R^1$ is a methyl group and $R^2$ is a residual group formed by removing a hydroxyl group from the telomer obtained in 1) above was obtained.

3) Preparation of a soft, putty-like material for lining a denture base

In the same manner as in 3) of Example 1 except for using 100 g of the monomer obtained in 2) above and 10 g of 2-ethyl hexyl acrylate instead of the monomer compound obtained in 2) of Example 1 and 15 g of methyl methacrylate of Example 1, still another soft, putty-like material according to the present invention was obtained, which had an interstitial resistance of 9.5 g/mm$^2$ before polymerization.

4) Preparation of a denture base lined with the soft, putty-like material according to the present invention

In the same manner as in 4) of Example 1 except for using the soft, putty-like material obtained in 3) above instead of that obtained in Example 1, still another denture base lined with the elastic and soft resin was obtained.

5) Determination of the elastic and soft resin lining

The Shore hardness (D) of the test piece made of the lining material obtained by 4) above was determined as in 5) of Example 1 and the Shore D was 24.

As a result of applying the denture base lined with the soft, putty-like material obtained in 4) above in an oral cavity of a 74 year old man, no deterioration of the denture base was observed even after 2 years of use thereof, and continued use thereof was possible.

Example 12

1) Preparation of a monomer represented by the formula (I)

In the same procedure as in 2) of Example 1 except for using 10 g of acryloyl chloride and the fluorine-containing telomer obtained in 1) of Example 10 instead of 12.5 g of methacryloyl chloride and the fluorine-containing telomer obtained in 1) of Example 1, a monomer represented by the formula (I) wherein $R^1$ is a hydrogen atom and $R^2$ is a residual group formed by removing a hydroxyl group from a telomer comprising 50 parts by weight of vinylidene fluoride, 30 parts by weight of chlorotrifluoroethylene and 20 parts by weight of hexafluoropropylene having a molecular weight of 1020 was obtained.

2) Preparation of a soft, putty-like material for lining a denture base

In the same manner as in 3) of Example 1 except for using the monomer obtained in 1) above, 15 g of ethylacrylate and 1 g of di-t-butyl peroxide as an initiator instead of the monomeric compound obtained in 2) 15 g of methyl methacrylate and 1 g of benzoyl peroxide as an initiator of Example 1, a soft, putty-like material for lining a denture base was prepared.

The interstitial resistance of the soft, putty material was 6.9 g/mm$^2$ before polymerization.

3) Preparation of a denture base lined with the soft, putty-like material according to the present invention

In the same manner as in 4) of Example 1 except for using the soft, putty-like material obtained in 2) above instead of that obtained in Example 1, a denture base according to the present invention lined with the elastic and soft resin was obtained.

4) Determination of the elastic and soft resin lining

The Shore hardness (D) of the test piece made of the lining material obtained in 3) above was determined as in 5) of Example 1 and the Shore D was 22.

As a result of applying the thus prepared denture base lined with the soft putty-like material obtained in 3) mentioned above in an oral cavity of a 76 year old woman no deterioration was observed even after 2 years of use thereof, and continued use thereof was possible.

## Claims

1. A composition suitable for use in lining a denture base, which composition comprises from 3 to 30 parts by weight of a fluorine-containing copolymer, from 3 to 30 parts by weight of a ($C_1$ to $C_{12}$-alkyl)acrylate or methacrylate and from 0.1 to 3.0 parts by weight of a polymerization-initiator per 100 parts by weight of a monomer represented by the formula (I):

$$CH_2{=}\underset{\displaystyle R^1}{\underset{|}{C}}{-}COOR^2 \qquad (I)$$

wherein $R^1$ represents a hydrogen atom or a methyl group and $R^2$ represents the group formed by removing a hydroxyl group from a hydroxyl group-containing and fluorine-containing telomer having a molecular weight of from 200 to 1500.

2. A composition according to Claim 1, wherein the hydroxyl group containing and fluorine-containing telomer has been obtained by subjecting a mixture of at least two monomers selected from vinylidene fluoride, vinyl fluoride, trifluoroethylene, chlorotrifluoroethylene, tetrafluoroethylene and hexafluoropropylene to telomerization.

3. A composition according to Claim 2, wherein the mixture contains not less than 50% by weight of vinylidene fluoride.

4. A composition according to Claim 2 or 3, wherein telomerisation was conducted in the presence of methanol as telogen.

5. A composition according to any one of the preceding Claims, wherein the telomer from which $R^2$ in formula (I) has been derived had a molecular weight of from 800 to 1200.

6. A composition according to any one of the preceding claims, wherein the fluorine-containing copolymer is derived from two or more monomers selected from vinylidene fluoride, vinyl fluoride, trifluoroethylene, chlorotrifluoroethylene, tetrafluoroethylene and hexafluoropropylene.

7. A composition according to Claim 6, wherein the fluorine-containing copolymer is a terpolymer of from 40 to 60% by weight of vinylidene fluoride and from 20 to 30% by weight of each of two of the tetrafluoroethylene, chlorotrifluoroethylene and hexaflouropropylene.

8. A composition according to any one of the preceding Claims, comprising from 5 to 20 parts by weight of the fluorine-containing copolymer per 100 parts by weight of monomer of formula (I).

9. A composition according to any one of the preceding claims, comprising from 5 to 20 parts by weight of ($C_1$ to $C_{12}$-alkyl)acrylate or methacrylate per 100 parts by weight of monomer of formula (I).

10. A method of lining a denture base, which method comprises lining the denture base with a composition as claimed in any one of the preceding Claims and polymerising the composition by heating.

**Patentansprüche**

1. Zusammensetzung, die zur Verwendung beim Auskleiden eines Gebißträgers bzw. einer Gebißprothese geeignet ist, welche 3 bis 30 Gewichtsteile eines fluorhaltigen Copolymers, 3 bis 30 Gewichtsteile eines $C_1$—$C_{12}$-Alkylacrylats oder -methacrylats und 0,1 bis 3,0 Gewichtsteile eines Polymerisationsinitiators pro 100 Gewichtsteile eines Monomers, dargestellt durch die Formel I

$$CH_2{=}\underset{\displaystyle R^1}{\underset{|}{C}}{—}COOR^2 \qquad (I)$$

umfaßt, worin
$R^1$ ein Wasserstoffatom oder eine Methylgruppe bedeutet und
$R^2$ die Gruppe bedeutet, die durch Entfernen einer Hydroxylgruppe aus einem hydroxylgruppenhaltigen und fluorhaltigen Telomer mit einem Molekulargewicht von 200 bis 1500 gebildet worden ist.

2. Zusammensetzung nach Anspruch 1, worin das hydroxylgruppenhaltige und fluorhaltige Telomer erhalten worden ist durch Aussetzen einer Mischung aus wenigstens zwei Monomeren, gewält aus Vinylidenfluorid, Vinylfluorid, Trifluorethylen, Chlortrifluorethylen, Tetrafluorethylen und Hexafluorpropylen, einer Telomerisation.

3. Zusammensetzung nach Anspruch 2, worin die Mischung nicht weniger als 50 Gew.-% Vinylidenfluorid enthält.

4. Zusammensetzung nach Anspruch 2 oder 3, worin die Telomerisation in Gegenwart von Methanol als Telogen durchgeführt wurde.

5. Zusammensetzung nach einem der vorhergehenden Ansprüche, worin das Telomer, aus dem $R^2$ in der Formel I abgeleitet worden ist, ein Molekulargewicht von 800 bis 1200 besitzt.

6. Zusammensetzung nach einem der vorhergehenden Ansprüche, worin das fluorhaltige Copolymer aus zwei oder mehreren Monomeren, gewählt aus Vinylidenfluorid, Vinylfluorid, Trifluorethylen, Chlortrifluorethylen, Tetrafluorethylen und Hexafluorpropylen, abgeleitet ist.

7. Zusammensetzung nach Anspruch 6, worin das fluorhaltige Copolymer ein Terpolymer aus 40 bis 60 Gew.-% Vinylidenfluorid und 20 bis 30 Gew.-% jedes von zweien aus Tetrafluorethylen, Chlortrifluorethylen und Hexafluorpropylen, ist.

8. Zusammensetzung nach einem der vorhergehenden Ansprüche, umfassend 5 bis 20 Gewichtsteile des fluorhaltigen Copolymer pro 100 Gewichtsteile des Monomers der Formel I.

9. Zusammensetzung nach einem der vorhergehenden Ansprüche, umfassend 5 bis 20 Gewichtsteile des $C_1$—$C_{12}$-Alkylacrylats oder -Methacrylats pro 100 Gewichtsteile des Monomers der Formel I.

10. Verfahren zum Auskleiden eines Gebißträgers bzw. einer Gebißprothese, bei dem die Gebißprothese mit einer Zusammensetzung nach einem der vorhergehenden Ansprüche ausgekleidet wird und die Zusammensetzung durch Erwärmen polymerisiert wird.

**Revendications**

1. Composition appropriée à l'utilisation dans le revêtement d'une base de dentier, laquelle composition comprend de 3 à 30 parties en poids d'un copolymère fluoré, de 3 à 30 parties en poids d'un acrylate ou méthacrylate d'alkyle en $C_1$ à $C_{12}$ et de 0,1 à 3,0 parties en poids d'un agent d'amorçage de la polymérisation pour 100 parties en poids d'un monomère représenté par la formule (I):

$$CH_2{=}\underset{\displaystyle R^1}{\underset{|}{C}}{—}COOR^2 \qquad (I)$$

dans laquelle $R^1$ représente un atome d'hydrogène ou un groupe méthyle et $R^2$ représente le groupe formé en éliminant un groupe hydroxyle d'un télomère hydroxylé et fluoré ayant une masse moléculaire de 200 à 1 500.

2. Composition suivant la revendication 1, dans laquelle le télomère hydroxylé et fluoré a été obtenu en soumettant un mélange d'au moins deux monomères choisis parmi le fluorure de vinylidène, le fluorure de vinyle, le trifluoréthylène, le chlorotrifluoroéthylène, le tétrafluoréthylene et l'hexafluoropropylène à une télomérisation.

3. Composition suivant la revendication 2, dans laquelle le mélange ne contient pas moins de 50% en poids de fluorure de vinylidène.

4. Composition suivant la revendication 2 ou 3, dans laquelle le télomérisation a été effectuée en présence de méthanol comme télogène.

5. Composition suivant l'une quelconque des revendications précédentes, dans laquelle le télomère à partir duquel a été obtenu le $R^2$ de la formule (I) avait une masse moléculaire de 800 à 1 200.

6. Composition suivant l'une quelconque des revendications précédentes, dans laquelle le copolymère

fluoré est obtenu à partir de 2 monomères ou davantage choisis parmi le fluorure de vinylidène, le fluorure de vinyle, le trifluoroéthylène, le chlorotrifluoréthylène, le tétrafluoréthylène et l'hexafluoropropylène.

7. Composition suivant la revendication 6, dans laquelle le copolymère fluoré est un terpolymère de 40 à 60% en poids de fluorure de vinylidène et de 20 à 30% en poids de chacun de deux des monomères tétrafluoréthylène, chlorotrifluoréthylène et hexafluoropropylène.

8. Composition suivant l'une quelconque des revendications précédentes, comprenant de 5 à 20 parties en poids du copolymère fluoré pour 100 parties en poids de monomère répondant à la formule (I).

9. Composition suivant l'une quelconque des revendications précédentes, comprenant de 5 à 20 parties en poids d'acrylate ou de méthacrylate d'alkyle en $C_1$ à $C_{12}$ pour 100 parties en poids de monomère répondant à la formula (I).

10. Procédé de revêtement d'une base de dentier, lequel procédé comprend le revêtement de la base de dentier avec une composition telle que revendiquée dans l'une quelconque des revendications précédentes et la polymérisation de la composition par chauffage.